# NOUVEAU FASCICULE DE BREVET EUROPEEN

(11) **EP 0 901 365 B2**
(45) Date de publication et mention de la décision concernant l'opposition: **28.09.2005**
(45) Mention de la délivrance du brevet: 18.04.2001
(21) Numéro de dépôt: 97925108.9
(22) Date de dépôt: 20.05.1997
(51) Int. Cl.: A61K 7/13

(54) **COMPOSITION DE TEINTURE DIRECTE CAPILLAIRE COMPRENANT UN POLYMERE RETICULE A MOTIFS ACRYLIQUES ET ACRYLATES D'ALKYLES EN C 10-C 30**
Zusammensetzung zur Direktfärbung von Haaren, die ein vernetztes Polymer mit Acryleinheiten und mit C10-C30-Alkylacryleinheiten enthält
DIRECT CAPILLARY DYEING COMPOSITION COMPRISING A CROSS-LINKED POLYMER WITH ACRYLIC AND ALKYL C 10-C 30 ACRYLATE UNITS

(30) Priorité: 23.05.1996 FR 9606430
(43) Date de publication de la demande: 17.03.1999
(73) Titulaire: L'OREAL, 75008 Paris (FR)
(72) Inventeur: MAUBRU, Mireille, F-78400 Chatou (FR)
(74) Mandataire: Dossmann, Gérard
(86) Numéro de dépôt international: PCT/FR1997/000885
(87) Numéro de publication internationale: WO 1997/044004

(56) Documents cités:
- EP-A- 0 410 393
- EP-A- 0 445 714
- WO-A-93/02655
- FR-A- 2 189 380
- FR-A- 2 234 277
- FR-A- 2 382 232

## Description

L'invention concerne une composition de teinture pour cheveux comprenant au moins un colorant direct particulier et au moins un polymère réticulé à motifs acryliques et à motifs acrylates d'alkyles en C₁₀-C₃₀.

Il est connu de teindre les fibres capillaires avec des compositions de teinture directe suivant un procédé dit de «coloration directe» qui consiste à appliquer sur les fibres, des molécules colorantes ayant une affinité pour lesdites fibres, à les laisser pauser puis à rincer les fibres. Les colorations qui en résultent sont des colorations temporaires ou semi-permanentes suivant la nature des interactions entre les colorants directs et la fibre capillaire, et leur désorption de la surface et/ou du coeur de la fibre.
Pour faciliter l'application de telles compositions de teinture sur les cheveux, éviter notamment qu'elles ne coulent sur le front et le visage ou en dehors du point d'application initialement choisi, pendant l'application ou au cours du temps de pause nécesssaire à la coloration, on augmente classiquement la viscosité des compositions au moyen d'acide polyacrylique réticulé (agent épaississant).
Cependant les compositions de teinture à base de colorants directs et d'acide polyacrylique réticulé ne s'avèrent plus suffisamment satisfaisantes au niveau de leurs propriétés tinctoriales après qu'elles ont été stockées un certain temps à une température plus basse que la température ambiante, par exemple en dessous de 10°C, et notamment aux environs de 4°C.
On observe ainsi que les compositions stockées dans de telles conditions engendrent une montée plus faible du colorant direct sur les cheveux et présentent donc un pouvoir tinctorial insuffisant.

La présente invention vise à résoudre le problème ci-dessus, c'est-à-dire à proposer un moyen permettant de préserver le pouvoir tinctorial de compositions de teinture renfermant un colorant direct particulier, susceptibles d'être stockées à basses températures, en particulier à des températures inférieures à 10°C.

Or, après de nombreuses recherches menées sur la question, la demanderesse vient maintenant de découvrir, qu'il est possible de préserver le pouvoir tinctorial des compositions de coloration directe particulière, si l'on ajoute à ces compositions, une quantité efficace d'un polymère réticulé à motifs acryliques et à motifs acrylates d'alkyles en C₁₀-C₃₀.
Même après des conservations plus ou moins prolongées à des températures inférieures à 10°C, et notamment voisines de 4°C, on obtient des compositions présentant un bon pouvoir tinctorial, et dont la montée sur les cheveux est très satisfaisante.

Cette découverte est à la base de la présente invention.

La présente invention a ainsi pour objet une composition cosmétique de teinture capillaire, du type comprenant, dans un support cosmétiquement acceptable approprié pour la teinture, au moins un colorant direct choisi parmi les colorants nitrés benzéniques; les nitropyridines, les colorants anthraquinoniques de formule 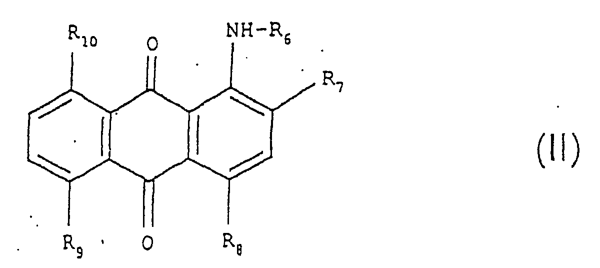 dans laquelle,
· R₆ désigne hydrogène, un radical monohydroxyalkyle ou polyhydroxyalkyle,
· R₇ désigne hydrogène, un radical alkyle ou alcoxy,
· R₈ désigne hydrogène, un radical hydroxy, amino, monohydroxyalkylamino ou polyhydroxyalkylamino,
· R₉ et R₁₀, identiques ou différents sont hydrogène, hydroxy ou amino,
étant entendu que les radicaux alkyles et alcoxy cités ci-avant sont en C₁-C₄ et qu'ils peuvent être linéaires ou ramifiés,
mono- ou di-azoïques, triarylméthaniques, aziniques, acridiniques et xanthéniques ou encore les colorants métallifères, et qui est caractérisée par le fait qu'elle comprend en outre au moins un polymère réticulé à motifs acryliques et à motifs acrylates d'alkyles en C₁₀-C₃₀.

La présente invention a également pour objet l'utilisation d'un polymère réticulé à motifs acryliques et à motifs acrylates d'alkyles en C₁₀-C₃₀ dans ou pour la fabrication d'une composition de teinture directe pour cheveux comprenant au moins un colorant direct, pour améliorer la conservation du pouvoir tinctorial de ladite composition, en particulier après des stockages en dessous de 10°C environ, et notamment aux environs de 4°C.

L'invention concerne aussi un procédé pour améliorer la conservation du pouvoir tinctorial, en particulier après des stockages en dessous de 10°C environ, et notamment aux environs de 4°C, d'une composition de teinture pour cheveux comprenant au moins un colorant direct, qui consiste à introduire dans ladite composition une quantité efficace d'au moins un polymère réticulé à motifs acryliques et à motifs acrylates d'alkyles en C₁₀-C₃₀.

Elle concerne enfin un procédé de teinture capillaire mettant en oeuvre les compositions à propriétés améliorées conformes à l'invention.

Mais d'autres caractéristiques, aspects, objets et avantages de l'invention apparaîtront encore plus clairement à la lecture de la description et des exemples qui suivent.

Selon l'invention, on entend désigner par motifs acryliques des motifs de structure 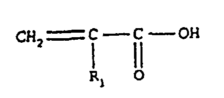 dans laquelle R₁ désigne H ou CH₃ ou C₂H₅, c'est-à-dire des motifs acide acrylique, acide méthacrylique ou acide éthacrylique.
On entend également désigner par motifs acrylates d'alkyles des motifs de structure : 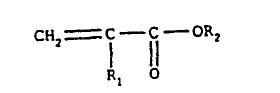 dans laquelle R₁ désigne H ou CH₃ ou C₂H₅, c'est-à-dire des motifs acrylates, méthacrylates ou éthacrylates, R₂ désignant un radical alkyle en C₁₀-C₃₀, de préférence en C₁₂-C₂₂.

Le ou les polymère(s) réticulé(s) à motifs acryliques et à motifs acrylates d'alkyles en C₁₀-C₃₀, utilisables dans le cadre de la présente invention, peuvent désigner plus particulièrement un terpolymère d'un mélange de monomères comprenant essentiellement :
(a) un acide acrylique, méthacrylique ou éthacrylique, mais de préférence acrylique ou méthacrylique,
(b) un acrylate de formule : 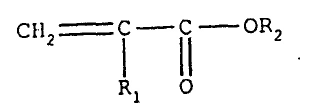 dans laquelle R₁ désigne H ou CH₃ ou C₂H₅, mais de préférence H ou CH₃, et R₂ désigne un radical alkyle ayant de 10 à 30 atomes de carbone et de préférence de 12 à 22 atomes de carbone, et,
(c) un monomère polymérisable réticulant, contenant un groupe CH₂=C〈avec au moins un autre groupement polymérisable dont les liaisons insaturées sont non conjuguées l'une par rapport à l'autre.

Des acrylates conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
Des monomères polymérisables réticulants du type (c) sont par exemple, et de préférence, des polyallyléthers tels que notamment le polyallylsucrose et le polyallylpentaérythritol.

Les polymères réticulés de ce type sont bien connus; ils sont préparés et décrits dans les brevets US-3 915 921 et 4 509 949.
Selon l'invention, on peut plus particulièrement utiliser (i) ceux qui sont constitués de 95 à 60% en poids de motifs acryliques, de 4 à 40% en poids de motifs acrylates et de 0,1 à 6% en poids de monomère réticulant de type (c), ou (ii) ceux qui sont constitués de 98 à 96% en poids de motifs acryliques, de 1 à 4% en poids de motifs acrylates et de 0,1 à 0,6% en poids de monomère réticulant de type (c).

Parmi lesdits polymères réticulés ci-dessus, on préfère tout particulièrement selon la présente invention, les produits vendus par la société GOODRICH sous les dénominations commerciales PEMULEN TR1, PEMULEN TR2, CARBOPOL 1342, et encore plus préférentiellement le PEMULEN TR1.

Les polymères réticulés à motifs acryliques et à motifs acrylates d'alkyles en C₁₀-C₃₀ décrits ci-dessus, sont utilisés dans la composition tinctoriale selon l'invention dans des proportions pouvant aller d'environ 0,05 à environ 5%, et de préférence d'environ 0,1 à environ 3% en poids par rapport au poids total de la composition.

Les colorants directs particuliers utilisables dans la composition tinctoriale selon la présente invention sont les colorants directs au sens défini ci-avant, c'est-à-dire utilisables suivant un procédé classique de coloration directe.
Parmi les colorants nitrés benzeniques utilisés, on peut citer les nitrophénylènediamines, les nitrodiphénylamines, les nitroanilines, les éthers de phénol nitrés ou les nitrophénols.

Les colorants directs plus particulièrement préférés selon l'invention sont choisis parmi les suivants :
**i)** les colorants nitrés benzéniques de formule (I) suivante : 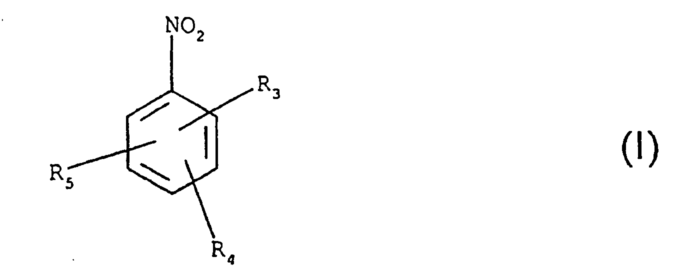 dans laquelle:
   - **R**_{**3**} désigne un radical NH₂, amino monosubstitué par un radical alkyle, monohydroxyalkyle, polyhydroxyalkyle, aminoalkyle, ou amino disubstitué par des radicaux, identiques ou différents, alkyle, mono- ou poly-hydroxyalkyle, ou aminoalkyle,
   - **R**_{**4**} désigne hydrogène, hydroxy, alcoxy, mono- ou poly-hydroxyalkyloxy, ou les mêmes significations désignées ci-dessus pour R₃, à l'exception du radical amino disubstitué,
   - **R**_{**5**} désigne hydrogène, alkyle, nitro, ou halogène,
**ii)** les colorants anthraquinoniques de formule (II) suivante : 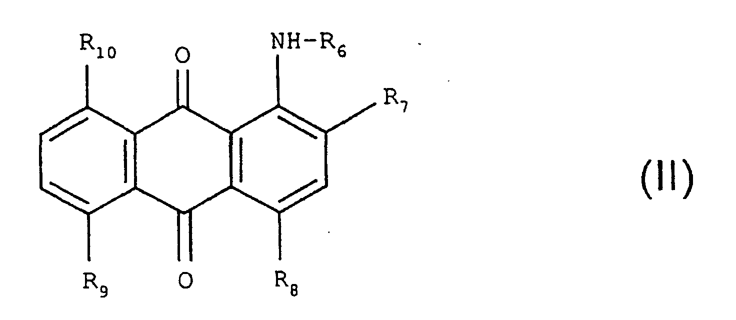 dans laquelle,
   - **R**_{**6**} désigne hydrogène, un radical monohydroxyalkyle ou polyhydroxyalkyle,
   - **R**_{**7**} désigne hydrogène, un radical alkyle ou alcoxy,
   - **R**_{**8**} désigne hydrogène, un radical hydroxy, amino ou monohydroxyalkylamino pu polyhydroxyalkylamino,
   - **R**_{**9**} et **R**_{**10**}, identiques ou différents sont hydrogène, hydroxy ou amino,

III) les colorants azoïques de formule (III) suivante : 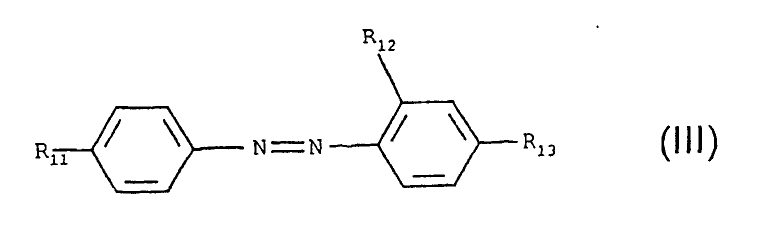 dans laquelle :
   - **R**_{**11**} désigne un radical nitro, amino, amino mono- ou di-substitué par des alkyles,
   - **R**_{**12**} désigne hydrogène ou un radical alkyle,
   - **R**_{**13**} désigne un radical amino, amino mono- ou di-substitué par des monohydroxyalkyles,
étant entendu que les radicaux alkyles et alcoxy cités ci-avant dans les formules (I), (II) et (III) sont en C₁-C₄ et qu'ils peuvent être linéaires ou ramifiés, et les sels cosmétiquement acceptables de tous ces composés.

Par C₁-C₄, on entend notamment les radicaux méthyle, éthyle, n-propyle, isopropyle, n-butyle, sec-butyle, et tert-butyle.
Par sels cosmétiquement acceptables, on désigne plus particulièrement les chlorhydrates, bromhydrates, et les sulfates.

Plus avantageusement encore, selon la présente invention, on préfère mettre en oeuvre les colorants directs suivants :
- 1-amino-2-nitro-4-N-(β-hydroxyéthyl)-amino-5-méthyl-benzène,
- 1,4,5,8-tétraaminoanthraquinone,
- 1,4-bis-N,N'-[(β,γ-dihydroxypropyl)-amino]-anthraquinone,
- 1,4,4-N-tris-(β-hydroxyéthyl)-1,4-diamino-2-nitro-benzène,
- 1-N-(β-hydroxyéthyl)-amino-2-nitro-4-amino-benzène,
- 1-hydroxy-3-nitro-4-amino-benzène,
- 1-hydroxy-3-nitro-4-N-(β-hydroxyéthyl)-amino-benzène,
- 1-β-hydroxyéthyloxy-3-méthylamino-4-nitro-benzène,
- 1-méthylamino-2-nitro-5-β,γ-dihydroxypropyloxy-benzène,
- 1 -N-(β-aminoéthyl)-amino-2-nitro-4-β-hydroxyéthyloxy-benzène,
- 4-[N-éthyl-N-(β-hydroxyéthyl)-amino]-1-N-(β-hydroxyéthyl)-amino-2-nitrobenzène,
- 1-(4'-amino-diphénylazo)-2-méthyl-4-N-bis-(β-hydroxyéthyl)-amino-benzène,
- 1-méthoxy-3-N-(β-aminoéthyl)-amino-4-nitro-benzène,
- 1-amino-2-nitro-4-N-(β-hydroxyéthyl)-amino-benzène,
- 1-amino-2-nitro-4-N-bis-(β-hydroxyéthyl)-amino-benzène,
- 1,4-N-bis-(β-hydroxyéthyl)-amino-2-nitro-benzène,
- 1-amino-2-N-(β-hydroxyéthyl)-amino-5-nitro-benzène,
- 1,4-diamino-anthraquinone,
et leurs sels cosmétiquement acceptables.

Ces colorants directs, sous forme de base ou salifiée, sont généralement présents dans la composition tinctoriale selon l'invention dans des proportions pouvant aller d'environ 0,001 à environ 10%, et de préférence d'environ 0,05 à environ 5% en poids par rapport au poids total de la composition.

Le milieu cosmétiquement acceptable pour la teinture est un milieu aqueux pouvant contenir un ou plusieurs solvants organiques choisi parmi les alcools comme par exemple l'alcool éthylique, l'alcool isopropylique, l'alcool benzylique et l'alcool phényléthylique, ou des glycols ou éthers de glycol, tels que par exemple, le propylèneglycol ou ses éthers tels que, par exemple, le monométhyléther de propylèneglycol, le butylèneglycol, le dipropylèneglycol, les éthers monométhylique, monoéthylique et monobutylique de l'éthylèneglycol, ainsi que les alkyléthers de diéthylèneglycol, comme par exemple, le monoéthyléther ou le monobutyléther du diéthylèneglycol, dans des proportions comprises entre environ 0,5 et 20% et, de préférence, entre environ 2 et 10% en poids par rapport au poids total de la composition.
On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.
On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.
Ladite composition tinctoriale peut en outre contenir divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des cheveux.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier par exemple de 3 à 12 et de préférence de 7 à 11 et plus préférentiellement encore de 8,5 à 10, et pouvant être ajusté au moyen d'agents d'alcalinisation ou d'agents d'acidification antérieurement bien connus. Comme agents alcalinisants, on peut citer l'ammoniaque, les carbonates alcalins, les alcanolamines, par exemple les mono- di- et triéthanolamines et leurs dérivés, les hydroxydes de sodium ou de potassium, et les composés de formule : 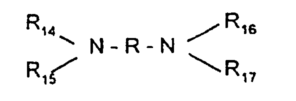 dans laquelle, R est un reste propylène éventuellement substitué par un groupement hydroxyle ou un radical alkyle en C₁-C₄ ; R₁₄ R₁₅, R₁₆ et R₁₇, simultanément ou indépendamment l'un de l'autre, représentent un atome d'hydrogène, un radical alkyle en C₁-C₄ ou hydroxyalkyle en C₁-C₄.

Les agents acidifiants sont classiquement des acides minéraux ou organiques comme par exemple les acides chlorhydrique, tartrique, citrique et phosphorique.

La composition appliquée sur les cheveux peut se présenter sous des formes diverses, telles que sous forme de liquide, de crème, de gel ou sous toute autre forme appropriée pour réaliser une teinture des cheveux. En particulier, elle peut être conditionnée sous pression en flacon aérosol en présence d'un agent propulseur et former une mousse.

Un autre objet de la présente invention porte sur un procédé de teinture des cheveux, par coloration directe, consistant à appliquer sur les cheveux secs ou humides, une composition tinctoriale telle que définie ci-avant, puis à laisser agir ladite composition de préférence pendant 3 à 60 minutes environ, à rincer les cheveux, puis à les laver éventuellement, à les rincer ensuite à nouveau, puis à les sécher.
On peut aussi laisser agir la composition, puis la sécher.

Des exemples concrets illustrant l'invention vont maintenant être donnés.

### EXEMPLE 1:

On a préparé la composition de teinture suivante :

| | |
|---|---|
| Colorant direct(1)* | 0,1 g |
| Alcool décylique oxyéthyléné par 5,3 moles d'oxyde d'éthylène | 2,0 g |
| Acide laurique | 1,0 g |
| Monobutyléther du diéthylèneglycol | 5,0 g |
| PEMULEN TR1 de Goodrich (copolymère réticulé acide | |
| acrylique/acrylates d'alkyles en C₁₀-C₃₀) | 0,51g |
| 2-amino-2-méthyl-1-propanol q.s pH | 9,5 |
| Eau déminéralisée q.s.p | 100 g |

| | |
|---|---|
| *colorant direct (1) : 1-amino-2-nitro-4-N-(β-hydroxyéthyl)amino-5-méthyl-benzène. | |

Après 24 heures, on a mesuré la viscosité de cette composition au viscosimètre Contrave à 25°C. La viscosité enregistrée était de 200 cp.
On a ensuite appliqué cette composition sur des mèches de cheveux gris naturels à 90% de blancs, et on a laissé pauser la composition pendant 30 minutes. Puis on a rincé les mèches à l'eau courante et on les a séchées.
Les mèches ont été teintes dans une nuance, qui chiffrée en valeur MUNSELL (Norme ASTM D 1535-68, laquelle définit la couleur : H, désignant la nuance ou Hue, V, désignant l'intensité ou Value, et C, désignant la pureté ou Chromaticité), sur un colorimètre MINOLTA CM 2002, était la suivante :
en H,V,C : **7,5 R 4,7 / 2,9.**
Lés mèches témoin (non teintes) présentaient une nuance H,V,C : **3,8Y 5,7 /1,6**.

On a également stocké la composition préparée ci-dessus pendant 1 mois à la température de 4°C.
Puis on a appliqué la composition ainsi conservée sur des mèches de cheveux de même qualité et suivant le même protocole que ci-dessus.
La nuance des mèches teintes au moyen de cette composition conservée à 4°C était la suivante :
en H,V,C : 7,9 R **4,7 / 2,8**.
Puis on a quantifié la modification de couleur entre les mèches teintes au moyen de la composition initiale et celles teintes au moyen de la composition conservée durant 1 mois, à la température de 4°C, en utilisant l'équation de NIC-KERSON qui définit les indices de variation de couleur :
I= (C/5) x 2ΔH + 6ΔV + 3ΔC (cette équation étant décrite dans la publication : «Journal of the Optical Society of America», 1944-sept-Vol34-n°9-p550-570).
Ainsi, la modification de coloration I_{b} (indice de variation de couleur entre des mèches teintes au moyen de la composition conservée 1 mois à la température de 4°C et celle des mèches teintes au moyen de la composition initiale) rapportée à la coloration initiale Iₐ (indice de variation de couleur des mèches teintes au moyen de la composition initiale et celle des mèches témoin), chiffrée en %, a été de 3,7%.

### EXEMPLE 2 COMPARATIF :

On a préparé une composition de teinture semblable à celle de l'exemple 1, de viscosité égale à celle de l'exemple 1, à base de polymère de l'art antérieur, en remplaçant simplement les 0,51 g de PEMULEN TR1 par 0,57 g de CARBOPOL 980 de la société Goodrich (acide polyacrylique réticulé de l'art antérieur-PM 4 000 000).
Des mèches de cheveux naturels à 90% de blancs ont été teintes au moyen de la composition initiale (i.e. avant conservation) et suivant un protocole identique à celui de l'exemple 1 en une nuance exprimée en H,V,C égale à : **8,1 R 4,9 / 2,9**.
Des mèches de cheveux naturels à 90% de blancs ont été teintes au moyen de la même composition mais conservée 1 mois à 4°C. La nuance obtenue a été :
H,V,C : **8,7 R 4,8** / **2,8.**
Le rapport I_{b}/Iₐ appliqué à cet exemple, et chiffré en %, a été de 8,5%.

### Conclusion :

Après conservation pendant 1 mois à 4°C, la composition de teinture de l'exemple 1 comprenant un polymère réticulé conforme à la présente invention, présente un pouvoir tinctorial très supérieur à celui de la composition de teinture de l'exemple 2 comprenant un polymère réticulé de l'art antérieur, puisque la dégradation qui est exprimée par le rapport I_{b} / Iₐ, chiffré en %, n'est que de 3,7% dans le cas de l'exemple 1, alors qu'elle est de 8,5% dans le cas de l'exemple 2.

### EXEMPLE 3 :

On a préparé la composition de teinture suivante :

| | |
|---|---|
| Colorant direct (2)* | 0,1 g |
| Alcool décylique oxyéthyléné par 5,3 moles d'oxyde d'éthylène | 2,0 g |
| Acide laurique | 1,0 g |
| Monobutyléther du diéthylèneglycol PEMULEN TR1 de Goodrich (copolymère réticulé acide | 5,0 g |
| acrylique/acrylates d'alkyles en C₁₀-C₃₀) | 0,54g |
| 2-amino-2-méthyl-1-propanol q.s. pH | 9,5 |
| Eau déminéralisée q.s.p. | 100 g |

| | |
|---|---|
| *colorant direct (2) : 1,4,5,8-tétraaminoanthraquinone (à 30% dispersée sur lignosulfate). | |

Après 24 heures, on a mesuré la viscosité de cette composition au viscosimètre Contrave à 25°C. La viscosité enregistrée était de 220 cp.
On a ensuite appliqué cette composition sur des mèches de cheveux gris permanentés à 90% de blancs, et on a laissé pauser la composition pendant 30 minutes. Puis on a rincé les mèches à l'eau courante et on les a séchées.
Les mèches ont été teintes dans une nuance, qui chiffrée en valeur MUNSELL, était la suivante, en H,V,C : **4,1 B 4,2 / 2,4**.
Les mèches témoin (non teintes) présentaient une nuance H,V,C : **4,4 Y 5,9 / 1,6.**

On a ensuite conservé ladite composition ci-dessus pendant 1 mois à la température de 4°C.
Puis on a appliqué la composition ainsi conservée sur des mèches de cheveux de même qualité et suivant le même protocole que ci-dessus.
La nuance des mèches teintes au moyen de cette composition conservée à 4°C était la suivante, en H,V,C : **2,1 B 4,4 / 2,2**.
Le rapport I_{b} (indice de variation de couleur entre des mèches teintes au moyen de la composition conservée 1 mois à la température de 4°C et celle des mèches teintes au moyen de la composition initiale) à la (indice de variation de couleur entre des mèches teintes au moyen de la composition initiale et celle des mèches témoin), chiffré en %, a été de 9,8%.

### EXEMPLE 4 COMPARATIF :

On a préparé une composition de teinture semblable à celle de l'exemple 3, de viscosité égale à celle de l'exemple 3, à base de polymère de l'art antérieur, en remplaçant simplement les 0,54 g de PEMULEN TR1 par 0,67 g de CARBOPOL 2984 de la société Goodrich (acide polyacrylique réticulé de l'art antérieur-PM 3 000 000).
Des mèches de cheveux permanentés à 90% de blancs ont été teintes au moyen de la composition initiale (i.e. avant conservation) et suivant un protocole identique à celui de l'exemple 3 en une nuance exprimée en H,V,C égale à : **5,4 B 4,1 / 3,1.**
Des mèches de cheveux permanentés à 90% de blancs ont été teintes au moyen de la même composition mais conservée 1 mois à 4°C. La nuance obtenue a été égale en H,V,C, à : **1,6 B 4,3 / 1,9.**
Le rapport I_{b} / Iₐ appliqué à cet exemple, et chiffré en %, a été de 22,9%.

### Conclusion :

Après conservation pendant 1 mois à 4°C, la composition de teinture de l'exemple 3 comprenant un polymère réticulé conforme à la présente invention, présente un pouvoir tinctorial très supérieur à celui de la composition de teinture de l'exemple 4 comprenant un polymère réticulé de l'art antérieur, puisque la dégradation qui est exprimée par le rapport I_{b} / Iₐ, chiffré en %, n'est que de 9,8% dans le cas de l'exemple 3, alors qu'elle est de 22,9% dans le cas de l'exemple 4.

### EXEMPLE 5 :

On a préparé la composition de teinture suivante :

| | |
|---|---|
| Colorant direct (3)* | 0,15 g |
| Alcool décylique oxyéthyléné par 5,3 moles d'oxyde d'éthylène | 2,0 g |
| Acide laurique | 1,0 g |
| Monobutyléther du diéthylèneglycol | 5,0 g |
| PEMULEN TR1 de Goodrich (copolymère réticulé acide acrylique/acrylates d'alkyles en C₁₀-C₃₀) | 0,52g |
| 2-amino-2-méthyl-1-propanolq.s.pH | 9,5 |
| Eau déminéraliséeq.s.p. | 100g |

| | |
|---|---|
| *colorant direct (3) : 1,4-bis-N,N'-[(β,γ-dihydroxypropyl)-amino]-anthraquinone. | |

Après 24 heures, on a mesuré la viscosité de cette composition au viscosimètre Contrave à 25°C. La viscosité enregistrée était de 210 cp.
On a ensuite appliqué cette composition sur des mèches de cheveux gris naturels à 90% de blancs, et on a laissé pauser la composition pendant 30 minutes. Puis on a rincé les mèches à l'eau courante et on les a séchées.
Les mèches ont été teintes dans une nuance, qui chiffrée en valeur MUNSELL, était la suivante, en H,V,C : **5,9 GY 5,1 /1,0.**
Les mèches témoin (non teintes) présentaient une nuance H,V,C : **3,8Y 5,7 / 1,6.**

On a ensuite conservé ladite composition ci-dessus pendant 1 mois à la température de 4°C.
Puis on a appliqué la composition ainsi conservée sur des mèches de cheveux de même qualité et suivant le même protocole que ci-dessus.
La nuance des mèches teintes au moyen de cette composition conservée à 4°C était la suivante, en H,V,C : **1,2 GY 5,1 /1,1**.
Le rapport I_{b} (indice de variation de couleur entre des mèches teintes au moyen de la composition conservée 1 mois à la température de 4°C et celle des mèches teintes au moyen de la composition initiale) à Iₐ (indice de variation de couleur entre des mèches teintes au moyen de la composition initiale et celle des mèches témoin), chiffré en %, a été de 16,6%.

### EXEMPLE 6 COMPARATIF :

On a préparé une composition de teinture semblable à celle de l'exemple 5, de viscosité égale à celle de l'exemple 5, à base de polymère de l'art antérieur, en remplaçant simplement les 0,52 g de PEMULEN TR1 par 0,65 g de CARBOPOL 2984 de la société Goodrich (acide polyacrylique réticulé de l'art antérieur).
Des mèches de cheveux naturels à 90% de blancs ont été teintes au moyen de la composition initiale (i.e. avant conservation) et suivant un protocole identique à celui de l'exemple 5 en une nuance exprimée en H,V,C égale à : **6,6 GY 5,2 / 1,0.**
Des mèches de cheveux naturels à 90% de blancs ont été teintes au moyen de la même composition mais conservée 1 mois à 4°C. La nuance obtenue a été égale en H,V,C, à : **10,0 Y 5,4/1,2.**
Le rapport I_{b} / Iₐ appliqué à cet exemple, et chiffré en %, a été de 34,2%.

### Conclusion :

Après conservation pendant 1 mois à 4°C, la composition de teinture de l'exemple 5 comprenant un polymère réticulé conforme à la présente invention, présente un pouvoir tinctorial très supérieur à celui de la composition de teinture de l'exemple 6 comprenant un polymère réticulé de l'art antérieur, puisque la dégradation qui est exprimée par le rapport I_{b} / Iₐ, chiffré en %, n'est que de 16,6% dans le cas de l'exemple 5, alors qu'elle est de 34,2% dans le cas de l'exemple 6.

## Revendications

1. Composition de teinture capillaire, du type comprenant, dans un support cosmétiquement acceptable approprié pour la teinture, au moins un colorant direct, choisis parmi les colorants nitrés benzéniques, les nitropyridines, les colorants anthraquinoniques de formule 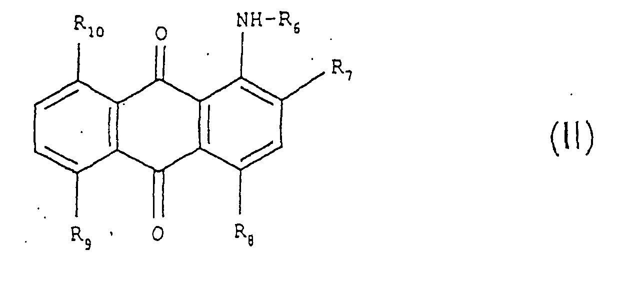 dans laquelle,
· R₆ désigne hydrogène, un radical monohydroxyalkyle ou polyhydroxyalkyle,
· R₇ désigne hydrogène, un radical alkyle ou alcoxy,
· R₈ désigne hydrogène, un radical hydroxy, amino, monohydroxyalkylamino ou polyhydroxyalkylamino,
· R₉ et R₁₀, identiques ou différents sont hydrogène, hydroxy ou amino,
étant entendu que les radicaux alkyles et alcoxy cités ci-avant sont en C₁-C₄ et qu'ils peuvent être linéaires ou ramlfiés,
mono- ou di-azoïques, triarylméthaniques, aziniques, acridiniques et xanthéniques ou en core les colorants métallifères,
**caractérisée par**le fait qu'elle comprend en outre au moins un polymère réticulé à motifs acryliques et à motifs acrylates d'alkyles en C₁₀-C₃₀.

2. Composition de teinture selon la revendication 1, **caractérisée par**le fait que le polymère réticulé est un terpolymère d'un mélange de monomères comprenant essentiellement :
(a) un acide acrylique, méthacrylique ou éthacrylique, de préférence acrylique ou méthacrylique,
(b) un acrylate de formule : 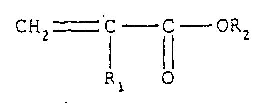 dans laquelle R₁ désigne H ou CH₃ ou C₂H₅, de préférence H ou CH₃, et R₂ désigne un radical alkyle ayant de 10 à 30 atomes de carbone, et,
(c) un monomère polymérisable réticulant, contenant un groupe CH₂=C〈 avec au moins un autre groupement polymérisable dont les liaisons insaturées sont non conjuguées l'une par rapport à l'autre.

3. Composition selon les revendications 1 ou 2, **caractérisée par le fait que** le radical alkyle du motif acrylate est en C₁₂-C₂₂.

4. Composition selon les revendications 1, 2, ou 3, **caractérisée par le fait que** l'agent réticulant est un polyallyléther.

5. Composition selon les revendications 1 à 4, **caractérisée par le fait que** le polymère réticulé est un terpolymère d'un mélange de monomères comprenant essentiellement :
(a) un acide acrylique,
(b) un acrylate de formule : 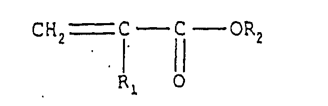 dans laquelle R₁ désigne H ou CH₃, et R₂ désigne un radical alkyle ayant de 12 à 22 atomes de carbone, et,
(c) un polyallyléther.

6. Composition selon l'une quelconque des revendications précédentes, **caractérisée par le fait que** le colorant direct est un colorant nitré benzénique de formule (I) suivante : 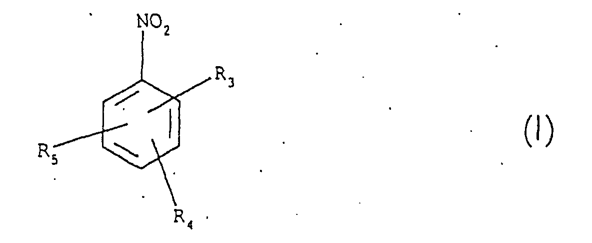 dans laquelle :
- R₃ désigne un radical NH₂, amino monosubstitué par un radical alkyle, monohydroxyalkyle, polyhydroxyalkyle, aminoalkyle, ou amino disubstitué par des radicaux, identiques ou différents, alkyle, mono- ou poly-hydroxyalkyle, ou aminoalkyle,
- R₄ désigne hydrogène, hydroxy, alcoxy, mono- ou poly-hydroxyalkyloxy, ou les mêmes significations désignées ci-dessus pour R₃, à l'exception du radical amino disubstitué,
- R₅ désigne hydrogène, alkyle, nitro, ou halogène,
étant entendu que les radicaux alkyles et alcoxy cités ci-avant sont en C₁-C₄ et qu'ils peuvent être linéaires ou ramifiés,
et les sels cosmétiquement acceptables de ces composés.

7. Composition selon la revendication 1, **caractérisée par le fait que** le colorant direct anthraquinonique est la 1,4-bis-N,N'-[(β,γ-dihydroxypropyl)-amino]anthraquinone.

8. Composition selon les revendications 1 à 5, **caractérisée par le fait que** le colorant direct est un colorant azoïque de formule (III) suivante : 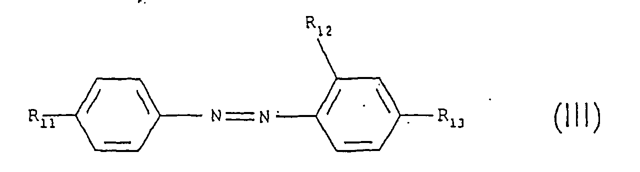 dans laquelle :
- R₁₁ désigne un radical nitro, amino, amino mono- ou di-substitué par des alkyles,
- R₁₂ désigne hydrogène ou un radical alkyle,
- R₁₃ désigne un radical amino, amino mono- ou di-substitué par.des monohydroxyalkyles,
étant entendu que les radicaux alkyles et alcoxy cités ci-avant sont en C₁-C₄ et qu'ils peuvent être linéaires ou ramifiés,
et les sels cosmétiquement acceptables de ces composés.

9. Composition selon les revendications 6 à 8, **caractérisée par** le lait que les sels cosmétiquement acceptables sont des chlorhydrates, des bromhydrates et des sulfates.

10. Composition selon l'une quelconque des revendications précédentes, **caractérisée par le fait que** le polymère réticulé est présent dans des proportions allant de 0,05 à 5% en poids, par rapport au poids total de la composition, et de préférence de 0,1 à 3%.

11. Composition selon l'une quelconque des revendications précédentes, **caractérisée par le fait que** le colorant direct est présent, sous forme de base ou salifiée, dans des proportions allant de 0,001 à 10% en poids, par rapport au poids total de la composition, et de préférence de 0,05 à 5%.

12. Composition selon l'une quelconque des revendications précédentes, **caractérisée par le fait que** le support cosmétiquement acceptable approprié pour la teinture est un support aqueux constitué par de l'eau et/ou des solvants organiques choisis parmi les alcools, les glycols et les éthers de glycol, dans des proportions comprises entre 0,5 et 20% en poids par rapport au poids total de la composition.

13. Utilisation d'un polymère réticulé tel que défini dans l'une quelconque des revendications 1 à 5, dans ou pour la fabrication d'une, composition de teinture directe pour cheveux comprenant au moins un colorant direct, pour améliorer la conservation du pouvoir tinctorial de ladite composition, en particulier après des stockages à basses températures.

14. Procédé pour améliorer la conservation du pouvoir tinctorial, en particulier après des stockages à basses températures, d'une composition de teinture contenant au moins un colorant direct, **caractérisé par le fait qu'**on ajoute à ladite composition, une quantité efficace d'un polymère réticulé tel que défini dans l'une quelconque des revendications 1 à 5.

15. Procédé de teinture des cheveux, par coloration directe, **caractérisé par le fait qu'**on applique une composition tinctoriale telle que définie à l'une quelconque des revendications 1 à 12 sur les cheveux secs ou humides, on laisse agir la composition, on rince les cheveux, puis éventuellement on les lave, et on les rince à nouveau, enfin on les sèche.

16. Procédé de teinture des cheveux, par coloration directe, **caractérisé par le fait qu'**on applique une composition tinctoriale telle que définie à l'une quelconque des revendications 1 à 12 sur les cheveux secs ou humides, on laisse agir la composition, puis on sèche les cheveux.

## Patentansprüche

1. Zusammensetzung zum Färben von Haaren, die in einem kosmetisch akzeptablen und zum Färben geeigneten Träger mindestens einen Direktfarbstoff enthält, der unter den nitrierten Benzolfarbstoffen, den Nitropyridinen, den Anthrachinonfarbstoffen der Formel: 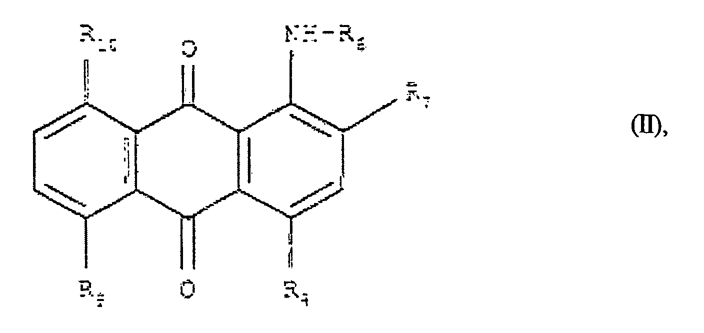 worin bedeuten:
- R₆ Wasserstoff, eine Monohydroxyalkylgruppe oder eine Polyhydroxyalkylgruppe,
- R₇ Wasserstoff, eine Alkylgruppe oder eine Alkoxygruppe,
- R₈ Wasserstoff, eine Hydroxygruppe, eine Aminogruppe, eine Monohydroxyalkylaminogruppe oder eine Polyhydroxyalkylaminogruppe und
- R₉ und R₁₀, die identisch oder voneinander verschieden sind, Wasserstoff, Hydroxy oder Amino,
mit der Maßgabe, dass die oben genannten Alkylgruppen und
Alkoxygruppen 1 bis 4 Kohlenstoffatome aufweisen und dass sie geradkettig oder verzweigt vorliegen können,
den Mono- oder Disazofarbstoffen, Triarylmethanfarbstoffen, Azinfarbstoffen, Acridinfarbstoffen und Xanthenfarbstoffen oder auch den metallhaltigen Farbstoffen ausgewählt ist, **dadurch gekennzeichnet, dass** sie ferner mindestens ein vernetztes Polymer mit Acryleinheiten und mit C₁₀₋₃₀-Alkylacrylateinheiten aufweist.

2. Zusammensetzung zum Färben nach Anspruch 1, **dadurch gekennzeichnet, dass** das vernetzte Polymer ein Terpolymer eines Gemisches von Monomeren ist, das im Wesentlichen umfasst:
(a) Acrylsäure, Methacrylsäure oder Ethacrylsäure und vorzugsweise Acrylsäure oder Methacrylsäure,
(b) ein Acrylat der Formel: 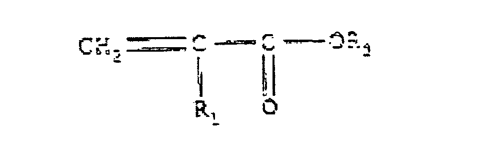 worin bedeuten:
- R₁ H, CH₃ oder C₂H₅ und vorzugsweise H oder CH₃ und
- R₂ eine Alkylgruppe mit 10 bis 30 Kohlenstoffatomen, und
(c) ein polymerisierbares vernetzendes Monomer, das eine Gruppe CH₂ = C und mindesten eine weitere polymerisierbare Gruppe enthält, deren ungesättigte Bindungen nicht konjugiert sind.

3. Zusammensetzung nach den Ansprüchen 1 oder 2, **dadurch gekennzeichnet, dass** die Alkylgruppe der Acrylateinheit 12 bis 22 Kohlenstoffatome aufweist.

4. Zusammensetzung nach den Ansprüchen 1, 2 oder 3, **dadurch gekennzeichnet, dass** das Vernetzungsmittel ein Polyallylether ist.

5. Zusammensetzung nach den Ansprüchen 1 bis 4, **dadurch gekennzeichnet, dass** das vernetzte Polymer ein Terpolymer eines Gemisches von Monomeren ist, das im Wesentlichen umfasst:
(a) Acrylsäure,
(b) ein Acrylat der Formel: 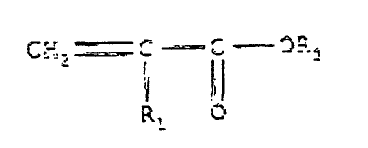 worin bedeuten:
- R₁ H oder CH₃ und
- R₂ eine Alkylgruppe mit 12 bis 22 Kohlenstoffatomen, und
(c) einen Polyallylether.

6. Zusammensetzung nach einem der vorhergehenden Ansprüche, **dadurch gekennzeichnet, dass** der Direktfarbstoff ein nitrierter Benzolfarbstoff der folgenden Formel (I): 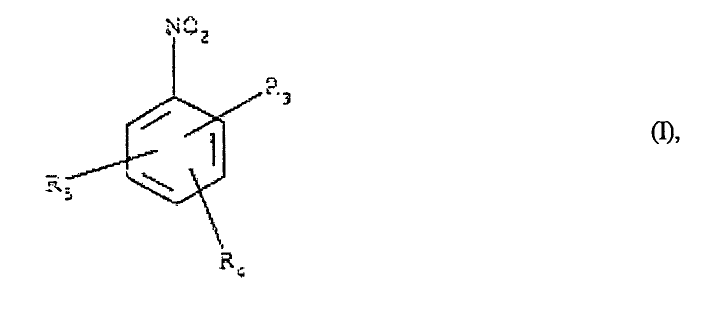 worin bedeuten:
- R₃ die Gruppe NH₂, eine einfach mit einer Alkylgruppe, einer Monohydroxyalkylgruppe, einer Polyhydroxyalkylgruppe oder einer Aminoalkylgruppe substituierte Aminogruppe oder eine zweifach mit Alkylgruppen, Monohydroxyalkylgruppen, Polyhydroxyalkylgruppen oder Aminoalkylgruppen, die identisch oder voneinander verschieden sind, substituierte Aminogruppe,
- R₄ Wasserstoff, Hydroxy, Alkoxy, Monohydroxyalkyloxy, Polyhydroxyalkyloxy oder die oben für R₃ genannten Gruppen mit Ausnahme der zweifach substituierten Aminogruppe und
- R₅ Wasserstoff, Alkyl, Nitro oder Halogen,
mit der Maßgabe, dass die oben genannten Alkylgruppen und Alkoxygruppen 1 bis 4 Kohlenstoffatome aufweisen und dass sie geradkettig oder verzweigt vorliegen können,
oder ein kosmetisch akzeptables Salz dieser Verbindungen ist.

7. Zusammensetzung nach Anspruch 1, **dadurch gekennzeichnet, dass** der Anthrachinon-Direktfarbstoff 1,4-Bis-N,N'-[(β,γ-dihydroxypropyl)-amino]-anthrachinon ist.

8. Zusammensetzung nach den Ansprüchen 1 bis 5, **dadurch gekennzeichnet, dass** der Direktfarbstoff ein Azofarbstoff der folgenden Formel (III): 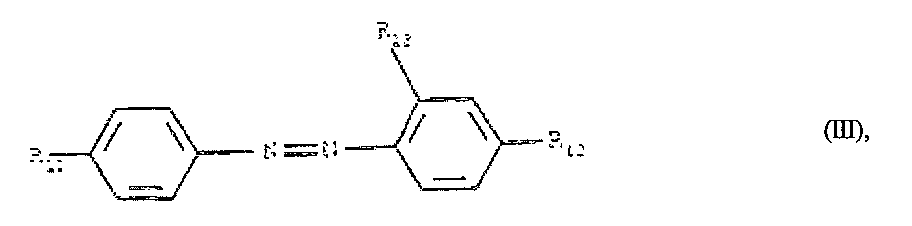 worin bedeuten:
- R₁₁ eine Nitrogruppe, eine Aminogruppe oder eine einfach oder zweifach mit Alkylgruppen substituierte Aminogruppe,
- R₁₂ Wasserstoff oder eine Alkylgruppe und
- R₁₃ eine Aminogruppe oder eine einfach oder zweifach mit Monohydroxyalkylgruppen substituierte Aminogruppe,
mit der Maßgabe, dass die oben genannten Alkylgruppen und Alkoxygruppen 1 bis 4 Kohlenstoffatome aufweisen und dass sie geradkettig oder verzweigt vorliegen können,
oder ein kosmetisch akzeptables Salz dieser Verbindungen ist.

9. Zusammensetzung nach den Ansprüchen 6 bis 8, **dadurch gekennzeichnet, dass** die kosmetisch akzeptablen Salze Chlorhydrate, Bromhydrate und Sulfate sind.

10. Zusammensetzung nach einem der vorhergehenden Ansprüche, **dadurch gekennzeichnet, dass** das vernetzte Polymer in Mengenanteilen im Bereich von 0,05 bis 5 Gew.-% und vorzugsweise von 0,1 bis 3 Gew.-%, bezogen auf das Gesamtgewicht der Zusammensetzung, vorliegt.

11. Zusammensetzung nach einem der vorhergehenden Ansprüche, **dadurch gekennzeichnet, dass** der Direktfarbstoff als solcher
oder in Form eines Salzes in Mengenanteilen im Bereich von 0,001 bis 10 Gew.-% und vorzugsweise von 0,05 bis 5 Gew.-%, bezogen auf das Gesamtgewicht der Zusammensetzung, vorliegt.

12. Zusammensetzung nach einem der vorhergehenden Ansprüche, **dadurch gekennzeichnet, dass** der kosmetisch akzeptable und zum Färben geeignete Träger ein wässeriger Träger ist, der aus Wasser und/oder organischen Lösemitteln besteht, die unter Alkoholen, Glykolen und Glykolethern ausgewählt sind und in Mengenanteilen im Bereich von 0,5 bis 20 Gew.-%, bezogen auf das Gesamtgewicht der Zusammensetzung, vorliegen.

13. Verwendung eines in einem der Ansprüche 1 bis 5 definierten vernetzten Polymers in einer Zusammensetzung zur Direktfärbung von Haaren, die mindestens einen Direktfarbstoff enthält, oder zu ihrer Herstellung, damit das Färbevermögen der Zusammensetzung insbesondere nach Aufbewahrung bei niedrigen Temperaturen besser erhalten bleibt.

14. Verfahren, um das Färbevermögen einer Färbemittelzusammensetzung, die mindestens einen Direktfarbstoff enthält, insbesondere nach Aufbewahrung bei niedrigen Temperaturen besser zu bewahren, **dadurch gekennzeichnet, dass** eine wirksame Menge eines in einem der Ansprüche 1 bis 5 definierten vernetzten Polymers zu der Zusammensetzung gegeben wird.

15. Verfahren zum Färben von Haaren durch Direktfärbung, **dadurch gekennzeichnet, dass** eine in einem der Ansprüche 1 bis 12 definierte Färbemittelzusammensetzung auf das trockene oder feuchte Haar aufgebracht wird, die Zusammensetzung dann einwirken gelassen wird, das Haar gespült, anschließend gegebenenfalls gewaschen und erneut gespült und schließlich getrocknet wird.

16. Verfahren zum Färben von Haaren durch Direktfärbung, **dadurch gekennzeichnet, dass** eine in einem der Ansprüche 1 bis 12 definierte Färbemittelzusammensetzung auf das trockene oder feuchte Haar aufgebracht wird, die Zusammensetzung dann einwirken gelassen wird und das Haar anschließend getrocknet wird.

## Claims

1. Composition for dyeing the hair, of the type comprising, in a cosmetically acceptable support which is suitable for dyeing, at least one direct dye, chosen from nitrobenzene dyes, nitropyridines, anthraquinone dyes of formula 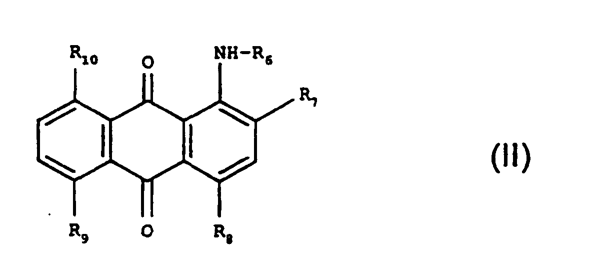 in which
- **R**_{**6**} denotes hydrogen or a monohydroxyalkyl or polyhydroxyalkyl radical,
- **R**_{**7**} denotes hydrogen or an alkyl or alkoxy radical,
- **R**_{**8**} denotes hydrogen or a hydroxyl, amino, monohydroxyalkylamino or polyhydroxyalkylamino radical,
- **R**_{**9**} and **R**_{**10**}**,** which may be identical or different, are hydrogen, hydroxyl or amino,
it being understood that the alkyl and alkoxy radicals mentioned above are C₁-C₄ and that they can be linear or branched, monoazo or diazo, triarylmethane, azine, acridine and xanthene dyes or alternatively metalliferous dyes, **characterized in that** it also comprises at least one crosslinked polymer containing acrylic units and C₁₀-C₃₀ alkyl acrylate units.

2. Dye composition according to Claim 1, **characterized in that** the crosslinked polymer is a terpolymer of a mixture of monomers essentially comprising:
(a) an acrylic, methacrylic or ethacrylic, but preferably acrylic or methacrylic, acid,
(b) an acrylate of formula: 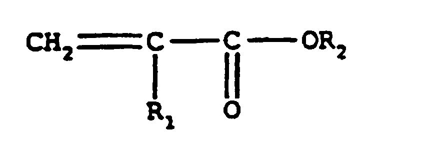 in which R₁ denotes H or CH₃ or C₂H₅, but preferably H or CH₃, and R₂ denotes an alkyl radical having from 10 to 30 carbon atoms, and,
(c) a crosslinking polymerizable monomer containing a CH₂=C〈 group with at least one other polymerizable group in which the unsaturated bonds are not conjugated with each other.

3. Composition according to Claim 1 or 2, **characterized in that** the alkyl radical of the acrylate unit is C₁₂-C₂₂.

4. Composition according to Claim 1, 2 or 3, **characterized in that** the crosslinking agent is a polyallyl ether.

5. Composition according to Claims 1 to 4, **characterized in that** the crosslinked polymer is a terpolymer of a mixture of monomers essentially comprising:
(a) an acrylic acid
(b) an acrylate of formula: 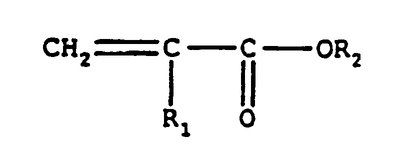 in which R₁ denotes H or CH₃, and R₂ denotes an alkyl radical having from 12 to 22 carbon atoms, and
(c) a polyallyl ether.

6. Composition according to any one of the preceding claims, **characterized in that** the direct dye is a nitrobenzene dye of formula (I) below: 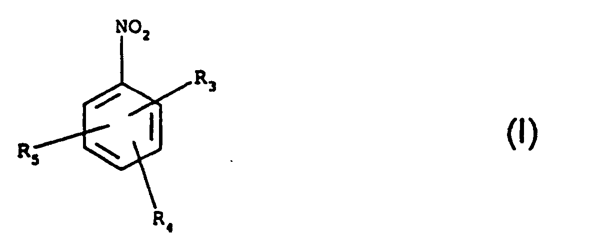 in which:
- **R**_{**3**} denotes an NH₂ radical, an amino radical monosubstituted with an alkyl, monohydroxyalkyl, polyhydroxyalkyl or aminoalkyl radical, or an amino radical disubstituted with identical or different alkyl, mono- or polyhydroxyalkyl or aminoalkyl radicals,
- **R**_{**4**} denotes hydrogen, hydroxyl, alkoxy, mono- or polyhydroxyalkyloxy, or the same meanings denoted above for R₃, except for the disubstituted amino radical,
- **R**_{**5**} denotes hydrogen, alkyl, nitro or halogen,
it being understood that the alkyl and alkoxy radicals mentioned above are C₁-C₄ and that they can be linear or branched,
and the cosmetically acceptable salts of these compounds.

7. Composition according to Claim 1, **characterized in that** the anthraquinone direct dye is 1,4-bis-N,N'-[(β,γ-dihydroxypropyl)amino]anthraquinone.

8. Composition according to Claims 1 to 5, **characterized in that** the direct dye is an azo dye of formula (III) below: 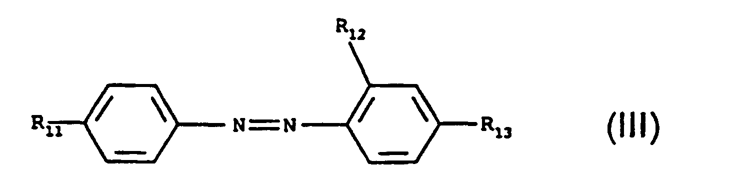 in which:
- **R**_{**11**} denotes a nitro or amino radical or an amino radical mono- or disubstituted with alkyls,
- **R**_{**12**} denotes hydrogen or an alkyl radical,
- **R**_{**13**} denotes an amino radical or an amino radical mono- or disubstituted with monohydroxyalkyls,
it being understood that the alkyl and alkoxy radicals mentioned above are C₁-C₄ and that they can be linear or branched,
and the cosmetically acceptable salts of these compounds.

9. Composition according to Claims 6 to 8, **characterized in that** the cosmetically acceptable salts are hydrochlorides, hydrobromides and sulphates.

10. Composition according to any one of the preceding claims, **characterized in that** the crosslinked polymer is present in proportions ranging from 0.05 to 5% by weight, relative to the total weight of the composition, and preferably from 0.1 to 3%.

11. Composition according to any one of the preceding claims, **characterized in that** the direct dye is present, in salified or base form, in proportions ranging from 0.001 to 10% by weight, relative to the total weight of the composition, and preferably from 0.5 to 5%.

12. Composition according to any one of the preceding claims, **characterized in that** the cosmetically acceptable support which is suitable for dyeing is an aqueous support consisting of water and/or organic solvents chosen from alcohols, glycols and glycol ethers, in proportions of between 0.5 and 20% by weight relative to the total weight of the composition.

13. Use of a crosslinked polymer as defined in any one of Claims 1 to 5, in, or for the manufacture of, a direct dye composition for the hair, comprising at least one direct dye, in order to improve the conservation of the dyeing power of the said composition, in particular after storage at low temperatures.

14. Process for improving the conservation of the dyeing power, in particular after storage at low temperatures, of a dye composition containing at least one direct dye, **characterized in that** an effective amount of a crosslinked polymer as defined in any one of Claims 1 to 5 is added to the said composition.

15. Process for dyeing the hair by direct dyeing, **characterized in that** a dye composition as defined in any one of Claims 1 to 12 is applied to wet or dry hair, the composition is left to act, the hair is rinsed, it is then optionally washed and is rinsed again and, lastly, it is dried.

16. Process for dyeing the hair by direct dyeing, **characterized in that** a dye composition as defined in any one of Claims 1 to 12 is applied to wet or dry hair, the composition is left to act and the hair is then dried.
